# EUROPÄISCHE PATENTSCHRIFT

(11) **EP 2 853 212 B1**
(45) Veröffentlichungstag und Bekanntmachung des Hinweises auf die Patenterteilung: **01.08.2018**
(21) Anmeldenummer: 14186153.4
(22) Anmeldetag: 24.09.2014
(51) Int. Cl.: A61B 17/88, A61B 17/70, A61B 17/86, A61B 90/00

(54) **Chirurgisches Instrument**
Surgical instrument
Instrument chirurgical

(30) Priorität: 30.09.2013 DE 102013110796
(43) Veröffentlichungstag der Anmeldung: 01.04.2015
(73) Patentinhaber: Z-Medical GmbH&Co. KG, 78532 Tuttlingen (DE)
(72) Erfinder: Paroth, Christel, 78532 Tuttlingen (DE); Henninger, Alexander, 78570 Mühlheim (DE); Combrowski, Zbigniew, 78532 Tuttlingen (DE)
(74) Vertreter: Patentanwälte und Rechtsanwalt Weiß, Arat & Partner mbB

(56) Entgegenhaltungen:
- US-A1- 2009 228 052
- US-A1- 2011 040 335
- US-A1- 2012 323 278
- US-A1- 2013 090 691
- US-A1- 2013 103 095

## Beschreibung

Die vorliegende Erfindung betrifft ein chirurgisches Instrument nach dem Oberbegriff des Anspruchs 1.

### Stand der Technik

In der Chirurgie werden Implantate, wie Cages, Schrauben od. dgl. eingesetzt. Dies ist insbesondere bei Wirbelsäulenoperationen der Fall.

Diese Implantate werden immer mehr mit einer Verlängerung, bspw. einem Schaft, Halte-/Einbringinstrument und einem Griff gefertigt, wobei die Verlängerung bspw. über eine Sollbruchstelle mit dem jeweiligen Implantat verbunden ist.

In diesem Zusammenhang wird auf die US 2009/0228052 A1 und die US 2012/0323278 A1, sowie der US 2013/0103095 A1 hingewiesen, welche jeweils ein chirurgisches Instrument offenbart, das über eine Verlängerung mit einer Abtrenneinrichtung verfügt.

Weiter wird auf die US 2011/0040335 A1 verwiesen, welche ein chirurgisches Instrument offenbart, bei dem eine Schwachstelle in der Aussenhülle als definierter Schwachpunkt zum Abbrechen ausgeführt ist.

Ausserdem wird auf die US 2013/0090691 A1 hingewiesen, welche ein Pedikelschrauben-Kit offenbart, bei dem ein Teil des Fixationsstabs an einem definierten Bruchpunkt abnehmbar ist.

Die Sollbruchstelle hat hierbei die Aufgabe, das Implantat mit der Verlängerung rigide zu halten, bis es in der gewünschten Position eingebracht ist.

Der Abriss der Verlängerung geschieht bei den meisten Herstellern durch Durchtrennen der Verlängerung in mehrere, meistens zwei Teile mittels entsprechenden Zusatzinstrumenten. Anschliessend wird die Verlängerung durch Hebelbewegungen von dem Implantat abgerissen. Dabei entstehen nachteilig Späne oder Splitter, welche wiederum das Infektionsrisiko erhöhen können. Der Abriss geschieht bei den meisten Herstellern durch die Verlängerung in mehrere, meistens zwei Teile mittels entsprechenden Zusatzinstrumenten.

### Aufgabe der Erfindung

Aufgabe der Erfindung ist es eine saubere, möglichst glatte und gratfreie Abbruchstelle zu erhalten und den Abriss splitterfrei und ohne grossen Kraftaufwand durchführen zu können. Dabei sollen auch Hebelbewegungen, die das umliegende Gewebe quetschen, ausweiten können verhindert werden.

### Lösung der Aufgabe

Zur Lösung der Aufgabe führen die Merkmale nach Anspruch 1. Vorteilhafte Weiterbildungen der Erfindung sind Gegenstand der abhängigen Ansprüche. Ein erfindungsgemäßes chirurgisches Instrument zum Abbruch einer Einbringhülse von einer mit der Einbringhülse verbundenen Einrichtung ist derart ausgestaltet, dass die Einbringhülse mit der medizinischen Einrichtung über eine Sollbruchstelle verbunden ist. Dies ist bspw. bei Pedikelschrauben der Fall. Dort werden Pedikelschrauben durch Pedikel in den Wirbelkörper eingeschraubt. Die Einbringhülse dient bei diesen minimal - invasiven Verfahren der Verlängerung einer Tulpe. Die Tulpe ist ein zwischen die Pedikelschraube und die Verlängerung geschaltetes Zwischenstück. Die Tulpe kann bspw. einen Stab aufnehmen, welcher verschiedene Pedikelschrauben intrakorperal verbindet und fixiert.

Hierzu weist der Ini auf der dem Stab abgewandten Seite bspw. eine Kreuzschlitzaufnahme für ein Schraubwerkzeug auf. Auf diese Weise kann durch die Einbringhülse hindurch der Ini mittels einer Schraubvorrichtung in die Ausnehmung der Tulpe hineingedreht werden, um einen dort eingebrachten Stab festzulegen.

Die Einbringhülse ist derart gestaltet, dass sie entweder direkt mit der Tulpe oder bspw. mit dem Schraubenkopf verbunden sein kann. Die Einbringhülse ist dabei zylindrisch und hohl, um andere Werkzeuge, wie bspw. die Schraubvorrichtung in die Einbringhülse geschoben werden kann, um die am Ende mit der Einbringhülse verbundene Einrichtung bspw. in einem Knochen festzuschrauben. Die im Rahmen der Erfindung angesprochene Einrichtung kann hierbei ein Cage, eine Schraube, insbesondere eine Knochenschraube oder andere Implantate bzw. intra- und/oder extrakorporale Verlängerungen dieser Implantate sein.

Das erfindungsgemäße chirurgische Instrument weist hierbei eine Verlängerung mit einem Abtrenner auf. Die Verlängerung ist hierbei ein Stabelement, welches entweder hohl oder voll ausgeführt werden kann. Das Stabelement kann insoweit auch linear oder gebogen oder flexibel ausgelegt sein. Letztendlich ist nur wichtig, dass das Stabelement dazu dient, den Abtrenner in den Bereich der Einbringhülse zu verbringen, an der die Einbringhülse von der mit der Einbringhülse verbundenen Einrichtung getrennt werden soll.

Der Abtrenner kann eine Lippe bzw. eine Nocke sein. Wichtig ist in diesem Zusammenhang lediglich, dass der Abtrenner bei bestimmungsgemässen Gebrauch in den Bereich der Einbringhülse gebracht werden soll, an den bspw. eine Sollbruchstelle zwischen der Einbringhülse und der mit der Einbringhülse verbundenen Einrichtung besteht.

Der Abtrenner kann als eine zumindest teilweise Kurve ausgebildet sein, wobei er eine Schneideform aufweisen kann. Es ist hierbei von Vorteil, wenn die Schneide scharf ist. Allerdings bedarf es keiner geschärften Schneide, um den Abtrennvorgang trotzdem durchzuführen.

Es ist denkbar, jedoch nicht erfindungsgemäß, dass die Verlängerung mit dem Abtrenner ein- oder mehrstückig ausgebildet ist. Bei einer einstückigen Ausbildung der Verlängerung mit dem Abtrenner bedarf es je nach Einbringhülse und Einrichtung verschiedener Arten von chirurgischen Instrumenten. Es ist aber auch denkbar, dass der Abtrenner bspw. über eine Schraubverbindung mit der Verlängerung verbindbar ist. In so einem Fall könnte die Verlängerung und der Abtrenner getrennt, also mehrstückig ausgebildet ist.

Auf diese Weise wäre es bspw. möglich, dass verschiedene Arten von Abtrennern auf ein und dieselbe Verlängerung aufgeschraubt werden könnten. Neben der Schraubverbindung des Abtrenners mit der Verlängerung sind aber andere Möglichkeiten der Verbindung denkbar, solange die Verbindung reversibel ist und verschiedene Arten von Abtrennern auf ein und dieselbe Verlängerung aufgebracht werden können.

Denkbar ist auch, dass das chirurgische Instrument ein Kombinationsinstrument ist, das mehrere Funktionen erfüllt. Bspw. Einschrauben und Abtrennen.

In einem nicht erfindungsgemäßen Ausführungsbeispiel weist die Verlängerung eine Kupplung zur Aufnahme eines Griffes auf. Die Kupplung ist hierbei andernends des Abtrenners an der Verlängerung ausgebildet. Nachdem der Griff auf die Kupplung aufgesetzt wurde, kann bspw. durch Drehen der Verlängerung der Abtrenner in eine bevorzugt rotierende Bewegung oder Richtung gebracht werden, so dass der Abtrenner durch seine Kurvenform eine schneidende, schälende, drückende Funktion ausführt und dabei die Einbringhülse von der medizinischen Einrichtung trennt. Der Abriss kann mit dem Instrument von Hand, also manuell oder durch einen geeigneten Antrieb erfolgen.

Die Verlängerung ist derart ausgebildet, dass die Verlängerung in die Einbringhülse eingebracht werden kann. Es ist aber auch denkbar, dass die Verlängerung derart ausgebildet ist, dass sie auf die Einbringhülse aufgesetzt werden kann. Dazu kann die Verlängerung bspw. durch Schlitze spreizbar sein, um die auf die Einbringhülse aufzusetzen und den Abtrenner an die betreffende Sollbruchstelle von aussen heranzuführen. Dieses Beispiel ist jedoch kein Teil der Erfindung. Als Aussen ist hierbei der Bereich zu verstehen, welcher nicht den Innenumfang der Einbringhülse darstellt.

Das erfindungsgemäße chirurgische Instrument ist derart ausgebildet, dass der Abtrenner vom Umfang der Verlängerung absteht. Dazu wird der Abtrenner in Form einer Nocke bzw. Lippe beispielsweise als Teil eines Kreises oder einer Zacke ausgebildet an den Umfang abstehend, d. h. im Wesentlichen rechtwinklig abstehend, angeformt.

Weiter ist der Abtrenner derart gestaltet, dass eine Abtrennung der Einbringhülse von der Tulpe eines Implantats oder des Implantats gewährleistet ist. Dabei weist der Abtrenner einerseits den Zapfen auf und andererseits die Verlängerung. Der Zapfen ist dabei exzentrisch angeordnet. Dies ist relativ zu der Verlängerung anzusehen. Bei einer nicht erfindungsgemäßen mittigen Anbringung des Zapfens stellt sich der Zapfen als lineare Fortführung der Verlängerung dar. Bei einer exzentrischen Ausführung wird der Zapfen nicht als gedachte lineare Fortführung, sondern als unterbrochene Fortführung ausgeführt. Dabei ist der Zapfen derart ausgebildet, dass er in ein Teil der Einrichtung einbringbar ist, wobei die Einrichtung als Rotationsgegenlager dient. Dabei kann das Teil bspw. ein Schraubenkopf oder eine Tulpe bzw. ein Ini, welcher in die Tulpe eingebracht ist, darstellen. Daraus folgt, dass der Zapfen zu einer besseren Führung bspw. in den Ini, die Tulpe oder den Schraubenkopf kraftschlüssig eingebracht werden kann. Es ist aber auch eine formschlüssige Verbindung denkbar. Es ist nicht zwingend erforderlich, dass eine kraftschlüssige Verbindung entsteht.

Der Zapfen dient als Rotationsgegenlager. Das bedeutet, dass eine Rotation bzw. Drehbewegung der Verlängerung in der Einbringhülse ermöglicht wird. Dazu kann der Zapfen entweder frei drehbar angeordnet sein oder beispielsweise den Ini mitdreht. Dies liegt im Ermessen des Fachmanns bzw. es kann an die Bedürfnisse des Nutzers angepasst werden.

### Figurenbeschreibung

Weitere Vorteile, Merkmale und Einzelheiten der Erfindung ergeben sich aus der nachfolgenden Beschreibung der Figuren. Diese zeigen in
- Figur 1: eine isometrische Seitenansicht eines erfindungsgemäßen chirurgischen Instrumentes;
- Figur 2: eine Vergrösserung eines Ausschnitts der Figur 1;
- Figur 3: eine isometrische Ansicht eines weiteren Ausführungsbeispiels für ein erfindungsgemäßes chirurgisches Instrument;
- Figur 4: eine vergrösserte Ansicht eines Teils der Figur 3;
- Figur 5: eine isometrische Ansicht eines anderen erfindungsgemäßen chirurgischen Instruments;
- Figur 6: eine vergrösserte Detailansicht eines Teils der Figur 5;
- Figur 7: eine teilwelse geschnittens isometrische Ansicht eines anderen erfindungsgemäßen Ausführungsbeispiels;
- Figur 8: eine vergrösserte Ansicht eines Teils der Figur 7;
- Figur 9: ein anderes erfindungsgemäßes Ausführungsbeispiel eines chirurgischen Instrumentes;
- Figur 10: eine vergrösserte Detailansicht der Figur 9.

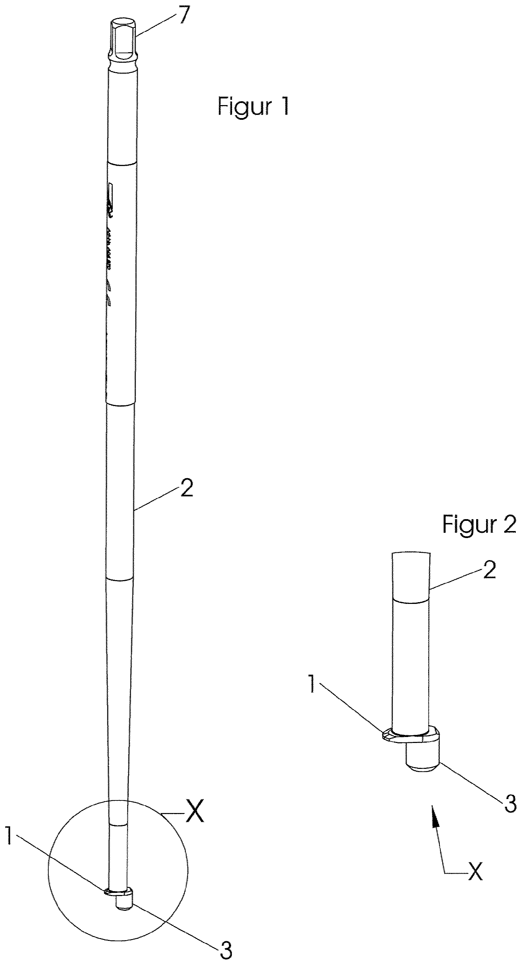

In der Figur 1 ist ein chirurgisches Instrument gezeigt. Das chirurgische Instrument besteht aus einer Verlängerung 2 und einem exzentrisch angeordneten Zapfen 3, welche einen Abtrenner 1 als Zwischenstück aufweist. Andernseits des Abtrenners 1 ist an die Verlängerung 2 eine Kupplung 7 angeformt. Ausserdem ist in der Figur 1 ein Vergrössungsausschnitt X gezeigt. Dieser Vergrösserungsausschnitt X ist in der Figur 2 nochmals dargestellt. Dort ist zu erkennen, wie der Zapfen 3 als unterbrochene Fortführung der Verlängerung 2 ausgebildet ist und zwischen dem Zapfen 3 und der Verlängerung 2 der Abtrenner 1 in Form eines Teilkreises schneidenförmig ausgebildet ist.

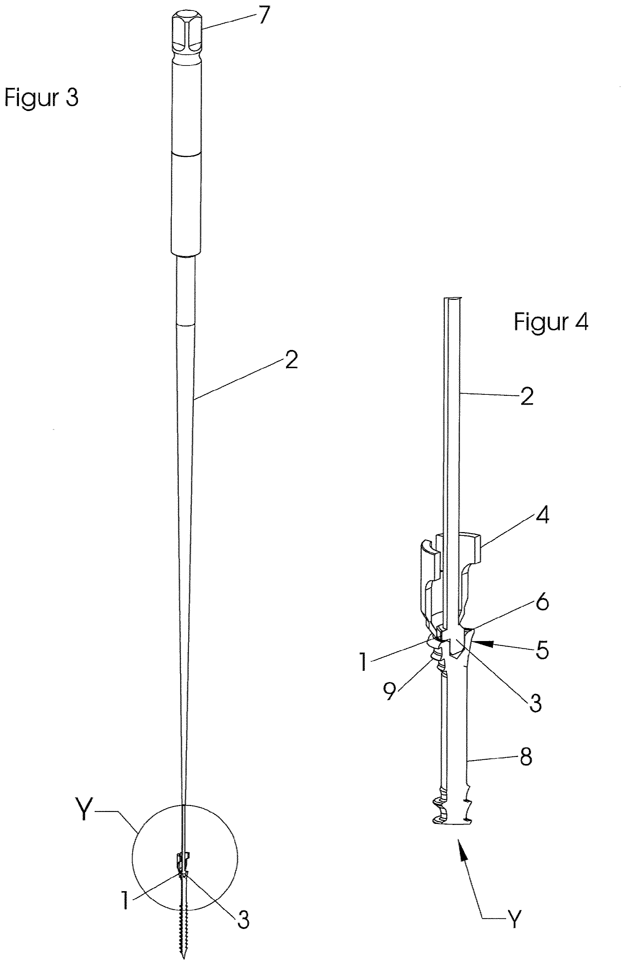

Die weiteren Ausführungen zu den Figuren 3 bis 10 sind derart zu lesen, dass die gleichen Merkmale mit den gleichen Bezugsziffern versehen werden. Die zu einer Bezugsziffer und dem dazugehörigen Merkmal gemachten Ausführungen geltend auch für die restlichen Figuren.

In Figur 3 ist die Verlängerung 2 mit der dazugehörigen Kupplung 7 gezeigt. Andernseits der Kupplung 7 formt die Verlängerung 2 wiederum einen Zapfen 3 und den Abtrenner 1 aus. Weiter ist in der Figur 3 ein Bereich Y gezeigt, welcher in der Figur 4 vergrössert dargestellt ist. In der Figur 4 ist auf Grund der teilweise geschnittenen Ansicht zu erkennen, wie der Zapfen 3 in eine Knochenschraube 8 eingreift. Dabei kann der Zapfen 3 kraft- und/oder formschlüssig mit der Knochenschraube verbunden werden.

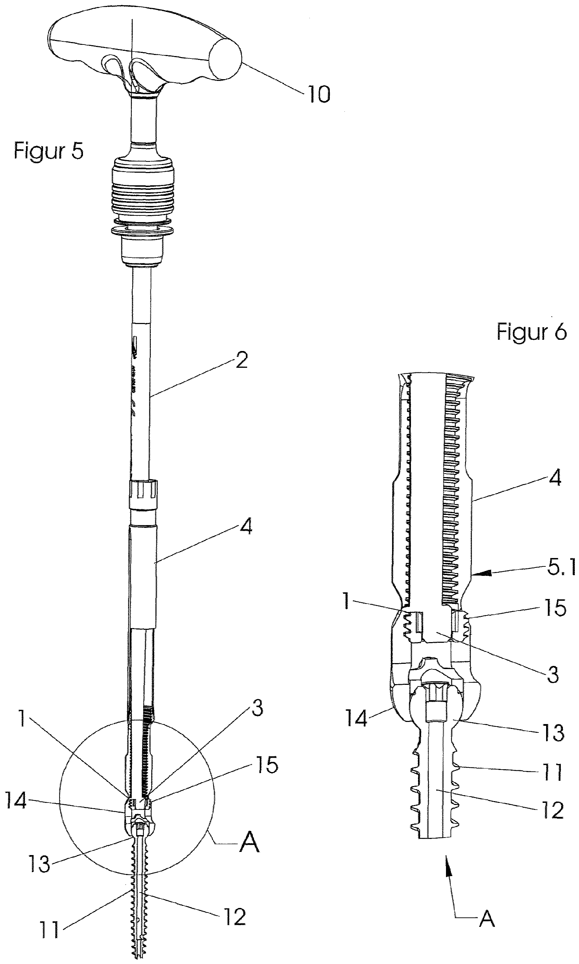

Der Unterschied zwischen einer kraftschlüssigen und einer formschlüssigen Verbindung besteht darin, dass bei einer kraftschlüssigen Verbindung die Knochenschraube 8 die Drehbewegung des Zapfens 3 mitmacht. Bei einer formschlüssigen Verbindung soll hier ein freies Drehen des Zapfens 3 in einem Schraubenkopf 9 möglich sein, ohne dass die Knochenschraube 8 die Drehbewegung der Verlängerung 2 nachvollzieht. Ausserdem ist die Einbringhülse 4 gezeigt, welche hier eine geringe Bauhöhe aufweist, als der Figur 5.

Der Abtrenner 1 kann mithilfe des exzentrisch angebrachten Zapfens 3 in dem Schraubenkopf ein Abtrennen der Einbringhülse 4 erreichen. Dazu wird die Verlängerung 2 um ihre eigene Achse gedreht. Diese Rotationsbewegung führt dazu, dass die Abtrenner 1 entlang des oberen Endes des Schraubenkopfes 9 geführt wird und dabei in einer definierten Bewegung die Einbringhülse 4 von dem Schraubenkopf 9 trennt, wobei die Einbringhülse 4 mit dem Schraubenkopf 9 über eine nicht näher beschriebene Sollbruchstelle verbunden ist.

In Figur 5 ist gezeigt, wie die Verlängerung 2 in die Einbringhülse 4 eingeschoben ist. Ausserdem ist ein Griff 10 gezeigt, welcher auf die nicht mehr zu erkennende Kupplung 7 aufgesetzt ist. Der Griff 10 ist derart mit der Verlängerung 2 verbunden, dass jede Rotationsbewegung des Griffs 10 auch eine Rotation der Verlängerung 2 nach sich zieht. Ein Teil der Figur 5 ist geschnitten dargestellt. In der Figur 5 ist nun eine Pedikelschraube 11 gezeigt, wobei diese Pedikelschraube 11 eine Kanulierung 12 und einen Pedikelschraubenkopf 13 aufweist. Dieser Pedikelschraubenkopf 13 ist in einer Tulpe 14 in einer definierten Weise beweglich angeordnet. Weiter ist in die Tulpe 14 ein Ini 15 eingeschraubt, in welchen wiederum der Zapfen 3 eingesetzt ist, welcher den Abtrenner 1 aufweist.

In der Figur 6 ist der Figur 5 als A bezeichnete Bereich vergrössert dargestellt. Dort ist gut zu erkennen, wie der Zapfen 3 in den Ini 15 eingebracht ist und der Abtrenner 1 derart angeordnet ist, als dass ein Abtrennen der Einbringhülse 4 von der Tulpe 14 erreicht werden kann.

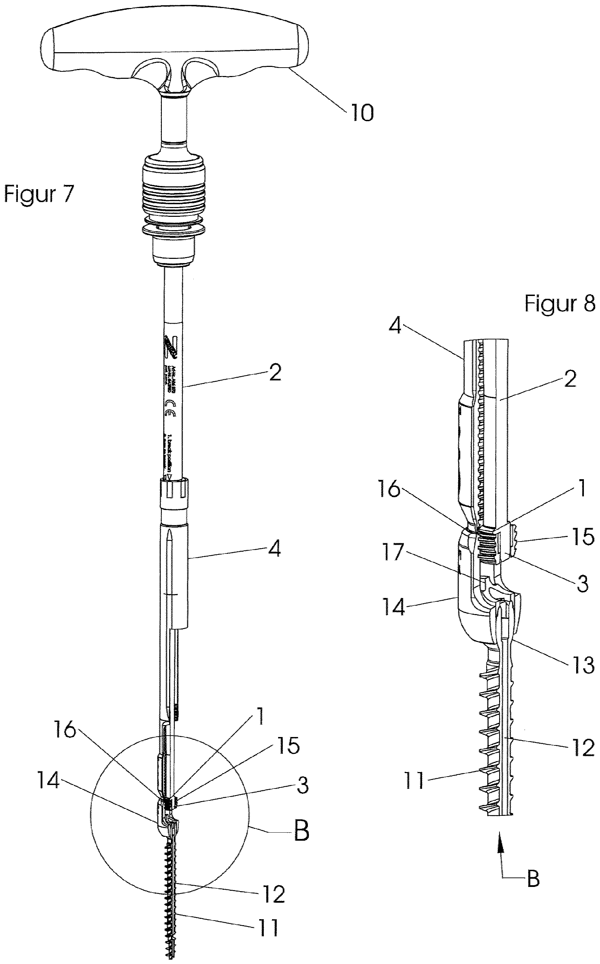

In der Figur 7 ist wiederum gezeigt, wie die Verlängerung 2 über die nicht gezeigte Kupplung 7 mit dem Griff 10 verbunden ist. Die Verlängerung 2 reicht in die Einbringhülse 4 hinein. Ein Teil der Figur 7 ist geschnitten dargestellt.

Ausserdem ist ein Bereich B gezeigt, welcher in der Figur 8 vergrössert dargestellt ist. In Figur 8 ist gut zu erkennen, wie der Zapfen 3 der Verlängerung 2 im Inneren der Einbringhülse 4 in den Ini 15 eingesetzt ist. Ausserdem ist zu erkennen, wie der Abtrenner 1 an eine Sollbruchstelle 16 anschlägt. Weiter ist gezeigt, wie die Tulpe 14 eine Öffnung 17 zur Aufnahme eines Stabs aufweist. Die Öffnung 17 kann bei einer Verbindung mit einer anderen Pedikelschraube 11 derart genutzt werden, als dass ein im Wesentlichen rechtwinklig zu der Pedikelschraube 11 angeordneter Stab durch die Öffnung 17 der Tulpe 14 greift und in eine andere Tulpe 14 und eine andere damit verbundene Partikelschraube 11 hineingreift. Anschliessend kann der Stab mittels Anziehen des Ini 15 in der Öffnung 17 der Tulpe 14 festgelegt werden.

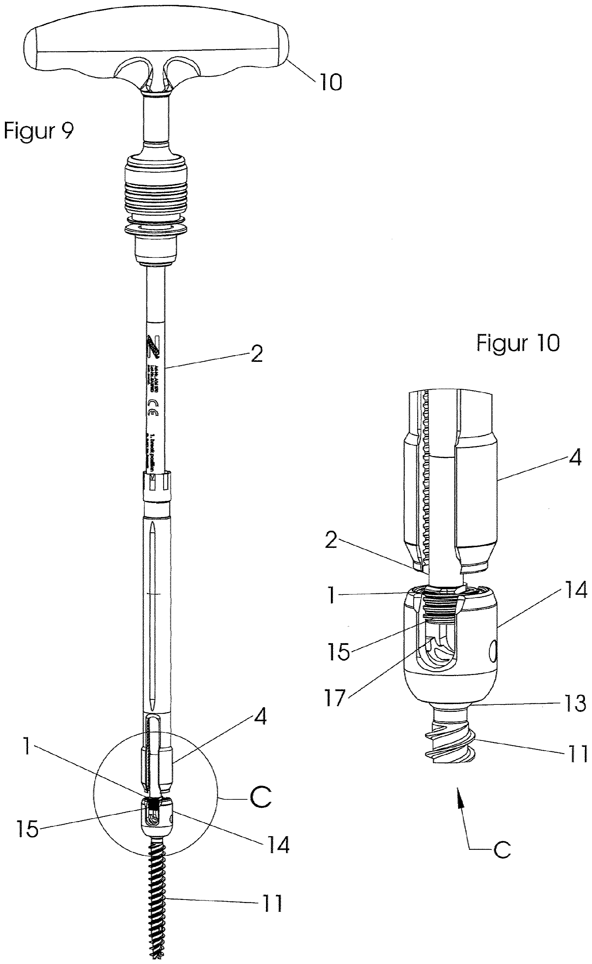

In den Figuren 9 und 10 ist gezeigt, wie die Einbringhülse 4 durch eine 360° Rotation des Abtrenners 1 an der Sollbruchstelle 16 von der Tulpe 14 abgetrennt ist. Dabei zeigt die Figur 10 einen vergrösserten Teilbereich C der Figur 9.

### Positionszahlenliste

| | | | | | |
|---|---|---|---|---|---|
| 1 | Abtrenner | 34 | | 67 | |
| 2 | Verlängerung | 35 | | 68 | |
| 3 | Zapfen | 36 | | 69 | |
| 4 | Einbringhülse | 37 | | 70 | |
| 5 | Einrichtung | 38 | | 71 | |
| 6 | Schraubenkopf | 39 | | 72 | |
| 7 | Kupplung | 40 | | 73 | |
| 8 | Knochenschraube | 41 | | 74 | |
| 9 | Schraubenkopf | 42 | | 75 | |
| 10 | Griff | 43 | | 76 | |
| 11 | Pedikelschraube | 44 | | 77 | |
| 12 | Kanulierung | 45 | | 78 | |
| 13 | Pedikelschraubenkopf | 46 | | 79 | |
| 14 | Tulpe | 47 | | | |
| 15 | Ini | 48 | | | |
| 16 | Soll bruchstelle | 49 | | | |
| 17 | Öffnung | 50 | | | |
| 18 | | 51 | | | |
| 19 | | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Chirurgisches Instrument zum Abbruch einer Einbringhülse von einer mit der Einbringhülse verbundenen Einrichtung (5, 5.1), die als Rotationsgegenlager dient, wobei das Instrument eine Verlängerung (2) mit einem Abtrenner (1) aufweist, wobei der Abtrenner (1) als ein Teilkreis oder eine Zacke auf dem Umfang der Verlängerung (2) ausgebildet ist,
**dadurch gekennzeichnet, dass**
der Abtrenner (1) einen Zapfen (3) umfasst, wobei der Abtrenner (1) einerseits den Zapfen (3) ausbildet und andererseits die Verlängerung (2) ausbildet,
wobei der Zapfen (3) in ein Teil der Einrichtung (5, 5.1) einbringbar ist, wobei der Zapfen (3) exzentrisch zu der Verlängerung (2) angeordnet ist.

2. Chirurgisches Instrument nach Anspruch 1, **dadurch gekennzeichnet, dass** die Verlängerung (2) in die Einbringhülse (4) einbringbar ist.

3. Chirurgisches Instrument nach Anspruch 1, **dadurch gekennzeichnet, dass** die Verlängerung (2) auf die Einbringhülse (4) aufsetzbar ist.

4. Chirurgisches Instrument nach einem der Ansprüche 1 bis 3, **dadurch gekennzeichnet, dass** das Teil, in den der Zapfen (3) einbringbar ist, ein Schraubenkopf (6) oder eine Tulpe (14) der Einrichtung (5, 5.1) ist.

## Claims

1. A surgical instrument for breaking off an introduction sleeve from a device (5, 5.1) connected to the introduction sleeve, which device serves as a rotation counter-surface, wherein the instrument has an extension (2) with a separator (1), wherein the separator (1) is designed as a partial circle or an indentation on the periphery of the extension (2),
**characterised in that**
the separator (1) comprises a peg (3), the separator (1) on one hand forming the peg (3) and on the other hand forming the extension (2),
the peg (3) being able to be introduced into a part of the device (5, 5.1), the peg (3) being arranged eccentrically to the extension (2).

2. A surgical instrument according to Claim 1, **characterised in that** the extension (2) can be introduced into the introduction sleeve (4).

3. A surgical instrument according to Claim 1, **characterised in that** the extension (2) can be placed on the introduction sleeve (4).

4. A surgical instrument according to one of Claims 1 to 3, **characterised in that** the part into which the peg (3) can be introduced is a screw head (6) or a tulip head (14) of the device (5, 5.1).

## Revendications

1. Instrument chirurgical pour casser un manchon d'insertion d'un moyen (5, 5.1) connecté au manchon d'insertion qui sert de contre-portée de rotation, l'instrument présentant une extension (2) avec un séparateur (1), dans lequel le séparateur (1) est réalisé sous forme de cercle partiel ou de pointe sur le pourtour de l'extension (2),
**caractérisé par le fait que**
le séparateur (1) présente un tourillon (3), où le séparateur (1) forme d'une part le tourillon (3) et d'autre part l'extension (2), le tourillon (3) pouvant être introduit dans une partie du dispositif (5, 5.1), le tourillon (3) étant disposé de manière excentrée par rapport à l'extension (2).

2. Instrument chirurgical selon la revendication 1, **caractérisé par le fait que** l'extension (2) peut être introduite dans le manchon d'insertion (4).

3. Instrument chirurgical selon la revendication 1, **caractérisé par le fait que** l'extension (2) peut être placé sur le manchon d'insertion (4).

4. Instrument chirurgical selon l'une des revendications 1 à 3, **caractérisé par le fait que** la partie dans laquelle peut être introduit le tourillon (3) est une tête de vis (6) ou une tulipe (14) du dispositif (5, 5.1).
